Europäisches Patentamt

European Patent Office

Office européen des brevets

(11) Publication number: **0 157 579**
A2

# (12) EUROPEAN PATENT APPLICATION

(21) Application number: 85302091.5

(22) Date of filing: 26.03.85

(51) Int. Cl.⁴: **B 01 L 3/00**, A 61 J 1/00

(30) Priority: 27.03.84 US 594027
18.10.84 US 662989

(43) Date of publication of application: 09.10.85
Bulletin 85/41

(84) Designated Contracting States: **AT BE CH DE FR GB IT
LI LU NL SE**

(71) Applicant: **INTERNATIONAL HEALTH SERVICES, 1898,
Bay Road, East Palo Alto California 94303 (US)**

(72) Inventor: **Stewart, David L., 568 7th St., Montaru ·
California (US)**
Inventor: **Nguyen, The Thanh, 947 Tamarack Lane,
No. 1 Sunnyvale California (US)**
Inventor: **Beal, C.B., 1312 Bellair Way, Menlo Park
California 94025 (US)**

(74) Representative: **Barlow, Roy James et al, J.A.KEMP &
CO. 14, South Square Gray's Inn, London WC1R 5EU
(GB)**

(54) **Specimen bag and injection assembly.**

(57) A specimen collection and analysis bag (1) with multiple
compartments (2, 3, 4) separated by frangible sealing means
(5) is provided. Improved septum means (7) for introducing the
specimen or other substances into the bag, whereby the sep-
tum is streched prior to injection, are disclosed. Additional
means (6, 8) for manipulating the contents of the individual
compartments, by expanding or compressing the latter, and for
guiding the use of the bag, including directionally biased fran-
gible sealing means, are taught.

- 1 -

## "SPECIMEN BAG AND INJECTION ASSEMBLY"

This invention relates in general to new and useful improvements in disposable specimen collection and analysis bags, and particularly in the manner in which the specimen is introduced into such bags. An important application of the present invention is in the form of clotting tests for blood.

Introduction of the specimen into a specimen collection and analysis device poses a number of problems. Use of an outright opening in the collection chamber necessitates the provision of either means for closing the opening directly, or means interposed between the opening and the actual area of collection to prevent backflow. Even while reliable, these means may considerably increase manufacturing costs.

The present invention provides a specimen bag comprising at least one chamber having an injection assembly whereby a substance may be injected into the collection chamber, said injection assembly comprising an elastic septum and means for stretching said septum.

Schwartz, US-A-3,660,033 (1972) describes a urine collection and analysis bag with a collection chamber, a reaction chamber, and reagent capsules held within the reaction chamber. The means for introducing the sample is a simple entrance, and the sample is held in the collection chamber by providing a sealing means in the form of an internal band of adhesive in the upper portion of the chamber.

If the specimen is directly injected into the compartment through a self-sealing septum, cf. Beacham, US-A-2,698,619, two problems must be considered. First, the natural elasticity of the septum may be insufficient to prevent leaks through the needle track. Second, the needle may inadvertently puncture the back of the bag, facing the septum, and thus cause leakage of the contents. (Barton, US-A-3,105,613 shows a blood bag with a self-sealing septum.)

It is known that one may utilize a tapering recess to guide a needle to a septum. McKinney, US-A-3,374,660; Halas, US-A-3,550,452, and Gianturco, US-A-4,445,896. This feature has not however, been combined with a septum-stretching means. It is also known that one may utilize a second septum to afford additional protection against leakage, see Tolbert, US-A-4,187,149. However, the use of a septum together with a penetrable but compressible cushion which holds a septum- piercing means away until compressive force is applied is not evident therefrom.

US-A-4,445,896 is of special interest in that it teaches that the self-sealing properties of the septum may be enhanced by maintaining it in a state of compression. (See also Martinez, US-A-3,814,137; Morgan, US-A-3,850,202; Lundquist, US-A-3,990,445.) Instead, applicants stretch the septum prior to penetration and allow it to relax after penetration.

In the field of gas chromatography, a puncturable, self-sealing septum is used to seal the inlet. Gas samples are injected through the septum, which heals when the needle is withdrawn. See, e.g., Tracht, US-A-2,839,152; McKinney, US-A-3,374,660; and Barre, US-A-3,537,321. The present invention, however, recites a novel injection assembly less liable to leakage upon repeated use than a simple septum.

Multicompartmented specimen collection or analysis bags with rupturable sealing means are known.

Steinberg, US-A-3,520,659 describes apparatus for determining the prothrombin time of a blood sample which includes prepackaged reagents in "top hat" reagent storage compartments, rather than in integrally formed compartments, which are easier to manufacture.

A number of blood collection devices are equipped with specimen treating means. Fox, US-A-2,690,179 describes a syringe equipped with a number of pods anchored to the inside wall of the collection chamber. The contents of these pods include anti-coagulants, preservatives, culture media, dyes and indicators. These pods are frangible.

Usually the structure of the seal is not disclosed. Known rupturable sealing means include weakened plastic walls, and sealing means cooperating with piercing means. See, e.g., Luhleich, US-A-3,552,441. The rupturable sealing means of the present invention, unlike the recited structures, encourages a gentle fluid flow upon rupture, while also favouring rupture in the desired direction.

Prenntzell, US-A-3,964,604 shows a flexible compartmented package with frangible sealing means in the form of a destructible "nose" extending from the primary or reaction chamber into the secondary or reagent chamber. These nose portions form a middle section in what is otherwise a permanent seam. The "nose" is ruptured by pressure on the secondary chamber (into which the nose extends) in order to expel material into the primary chamber. This differs substantially from the preferred directional seals of the present invention, in which the frangible seal is substantially linear and oblique permanent seals direct the pressure against it, said oblique seals forming a trapezoid pointing in the desired direction of flow. Prenntzell's "nose" points away from that direction.

Preferably the bag of the present invention includes a plurality of chambers with adjacent communicating chambers separated by independently frangible seals which, more preferably, are trapezoidal. Such trapezoidal frangible seals are to be preferred to Prenntzell's triangular seal which is likely to break at the point, resulting in "jetting" rather than in a smooth flow. Our trapezoidal seal experiences a larger break, diminishing "jetting".

Moreover, the art does not teach the utilization of directionally frangible reagent or diluent retaining means which rupture smoothly and easily in the preferred direction.

Shmelkin, US-A-4,365,727 describes a device employing two hinged frames to squeeze contents from a tube. The frames are not adhesive and therefore cannot be used to expand as well as compress the tube.

The invention is a substantially flat, disposable, flexible, transparent plastic pouch, preferably formed of two main sheets, for specimen collection and analysis (and particularly for medical diagnosis), having a collection or viewing chamber, a reagent chamber, and a diluent chamber, separated by frangible sealing means that rupture rapidly but smoothly. The preferred frangible sealing means may be directionally biased so reagents are released in the proper sequence. The collection chamber preferably receives specimens through an injection assembly which, by stretching a septum prior to injection of the specimen into the chamber, facilitates the healing of the septum after withdrawal of the needle.

To facilitate use of the specimen bag of this invention by less sophisticated personnel, it is desirable that the design make it easier to mix components in the desired sequence.

This can be done by placing the components in sequential order in sequential compartments and releasing them sequentially by rolling the device, or by utilizing directionally biased frangible seal betwixt the compartments, or both.

It is an object of the invention to provide a specimen collection and analysis bag having improved means for introducing the specimen or other substances into the bag.

It is desirable for the injector to be protected from contamination by the contents of a viewing chamber of the bag.

- 5 -

Adhesive material may be provided at the opposing hard surfaces around the compartments of the bag to adhere together the opposing hard surfaces to maintain the compartment collapsed to prevent liquid from returning to a compartment after having been expelled.

Ideally the bag should include a simple, easy to use and inexpensive means of rapidly but smoothly rupturing a frangible seal between compartments, encouraging nearly all fluid contained in one compartment to be moved into one or more adjacent compartments.

In order that the present invention may more readily be understood the following description is given, merely by way of example, with reference to the accompanying drawings, in which:-

FIGURE 1 is a schematic frontal view of a first embodiment of specimen bag according to the invention;

FIGURE 2 is a sectional view, taken along a line somewhat to one side of the midline of Figure 1;

FIGURE 3 is a detail perspective view of the injection assembly;

FIGURE 4 is a perspective view showing the manner in which the diluent is forced into the reagent chamber;

FIGURE 5 is a perspective view showing the manner in which the device is secured after the diluted reagent is forced into the collection chamber;

FIGURE 6 is a perspective view showing how the injection assembly is manipulated to facilitate specimen injections;

FIGURE 7 is a perspective view showing the observation of fibrin web formation within the collection chamber;

FIGURE 8 is a frontal view of a second embodiment;

FIGURE 9 is a sagittal section thereof;

FIGURE 10 is a cross-sectional view thereof, taken at the level of the back plate;

FIGURE 11 is a frontal view of a third embodiment;

FIGURE 12 is a sagittal view thereof;

FIGURE 13 is a frontal view of a fourth embodiment;

FIGURE 14 is a sagittal view thereof;

FIGURE 15 is a frontal (a) and sagittal (b) view of a modified injection assembly; and

FIGURE 16 is a sagittal view of a fourth embodiment.

FIGURE 17 is a schematic view of a fifth embodiment; and

FIGURE 18 presents views of three embodiments of the preferred directional frangible sealing means.

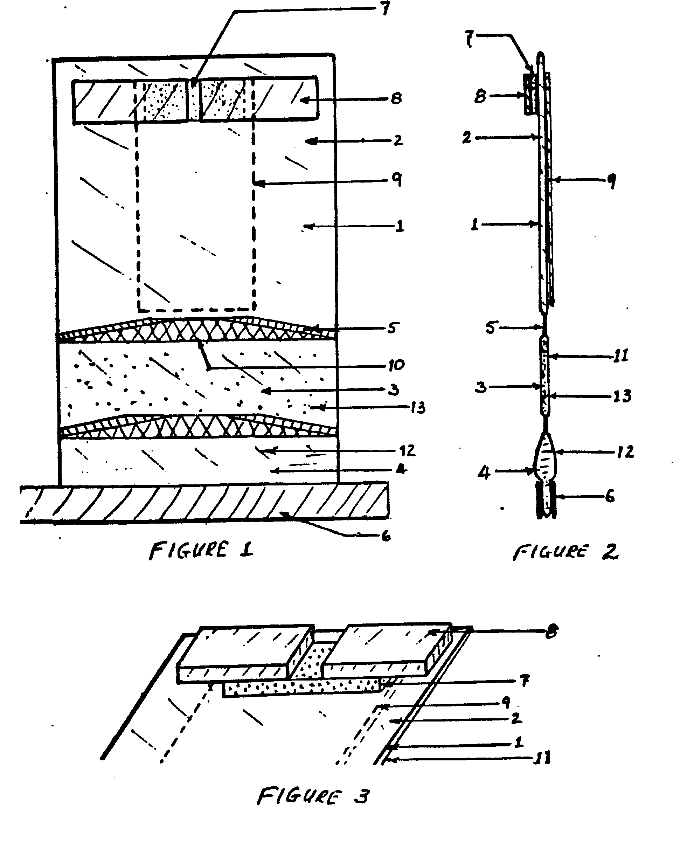

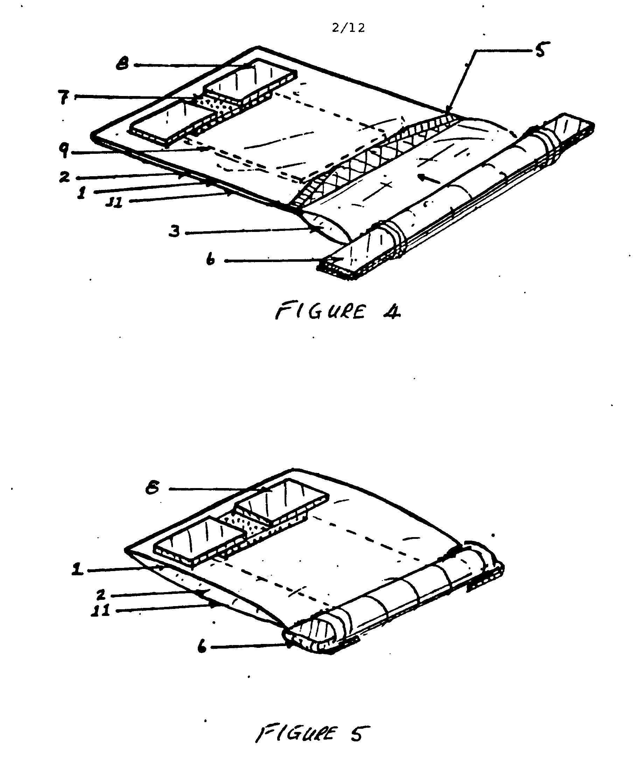

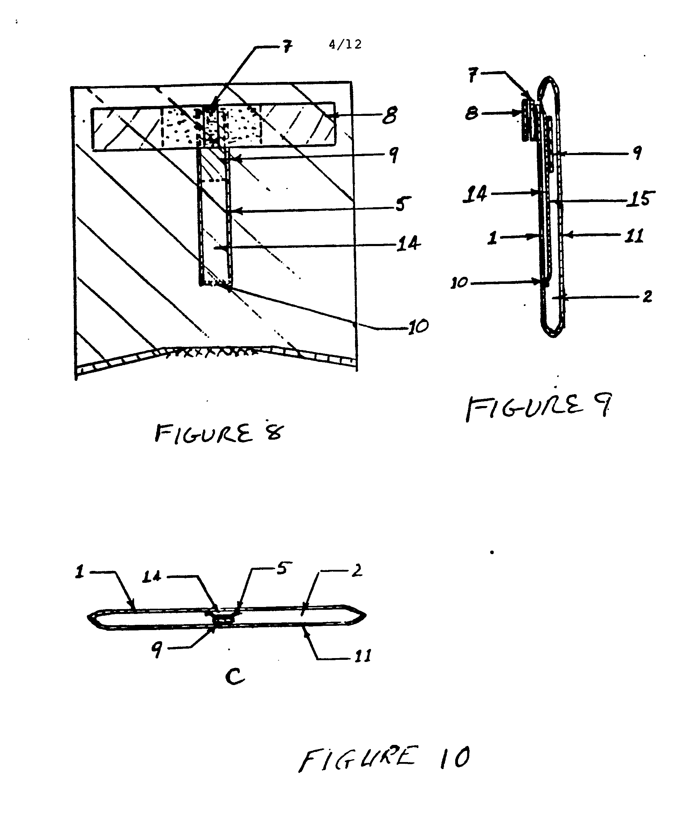

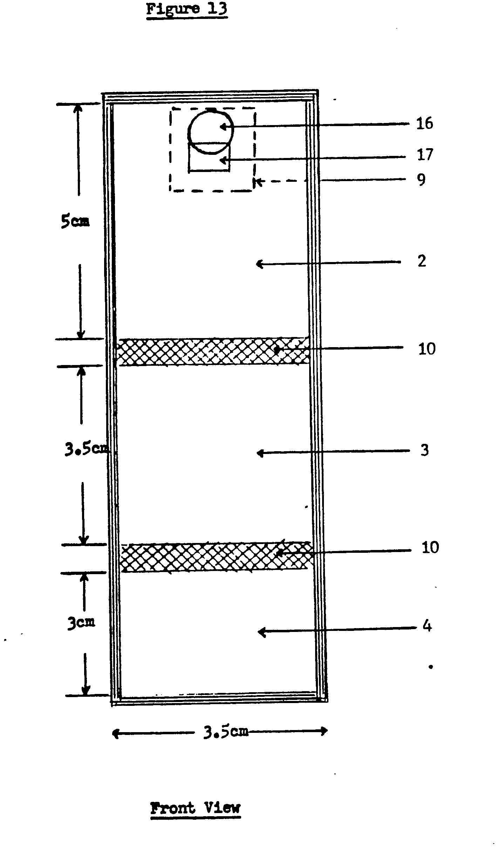

The device in its preferred embodiment is best understood by referring to the illustrations, in which Figure 1 is a schematic front view of the device, Figure 2 is a longitudinal section to one side of the mid-line, Figure 3 is a perspective of the injection assembly, and Figures 4-7 illustrate the use of the device.   Figures 8-17 illustrate additional embodiments.

The device is a thin, rectangular closed pouch approximately 5 cm. in width and 16 cm. in length, formed by sealing the edges of a front leaf 1 to a back leaf 11.  Of course, other dimensions may be selected. The front leaf 1 and back leaf 11 are preferably about 0.005 inch (0.1270 mm) in thickness. The front leaf is formed, at least in part, of a substantially transparent material, and preferably is made of a plastic such as polyvinyl chloride or the film known by the Trade Mark Surlyn.  To the upper part of the front leaf of the pouch is attached the injection assembly, consisting of a pliable latex membrane or septum 7 and two rigid lever means 8 attached to the latex membrane about 3 mm. lateral to the midline of the membrane, on opposite ends, as shown in Figure 3. To the upper part of the back leaf is attached a rectangular transparent rigid backing means (back plate) 9 which is appropriately 6 cm. in length and 2 cm. in width. Preferably, the edges of this back plate are so positioned as to act as fulcrum means

- 7 -

for the operation of the lever means 8.  (However,
the operator's finger may also serve as a fulcrum.)
The back plate may be located either within or without
the viewing chamber.  This backing means 9 may be
a simple rectangular frame, but preferably is a solid
plate.  If located within the collection chamber,
it acts to shield the back leaf from a needle piercing
the membrane 7.  For manufacturing reasons, it may
be placed outside the chamber, where it will still
prevent leakage. On the bottom part of the pouch is a rolling and

securing means, preferably a strip of adhesive-backed aluminum foil 6 attached to each leaf. These strips extend beyond the lateral ends of the pouch, and over this extension adhere to one another. The pouch is divided into three compartments. The upper and largest compartment is the collection or viewing chamber 2. This chamber is separated from the middle compartment, the dry component chamber 3 by a frangible seal 10, preferably of trapezoidal shape. This shape facilitates the smooth rupture of the seal in the desired direction. Preferably, above each descending margin of the frangible seal is a permanent seal 5, which also acts to directionally bias the seal. Similar seals divide the middle from the lower compartment, the diluent chamber 4. The reagent chamber contains reagent, such as dry 50 mg. of thromboplastin with a calcium salt 13, and the diluent chamber contains a diluent, such as 2 ml. of buffered saline diluent 12.

The device may be used to measure the prothrombin time of a specimen of whole blood or plasma. By producing a blood clot, the first element of which is the occurrence of a filmy fibrin web most readily visualized at the edges of the blood-diluent mixture. Preferably, the amount of thromboplastin used is slightly in excess of the total amount needed to maximize the rapidity of the reaction. The thromboplastin is preferably segregated from the diluent during storage because while dry thromboplastin is chemically stable, in solution thromboplastin is unstable, deteriorating within a few hours. Calcium ion is preferably added to the thromboplastin so that the reaction rate will not be dependent on calcium concentration in the test sample. The diluent serves to dissolve the thrombo-plastin, and to dilute the blood that is to be tested. The blood dilution retards the clotting reaction and lengthens by a few seconds the time interval between the visible formation of the fibrin web and the actual visible clot formation.

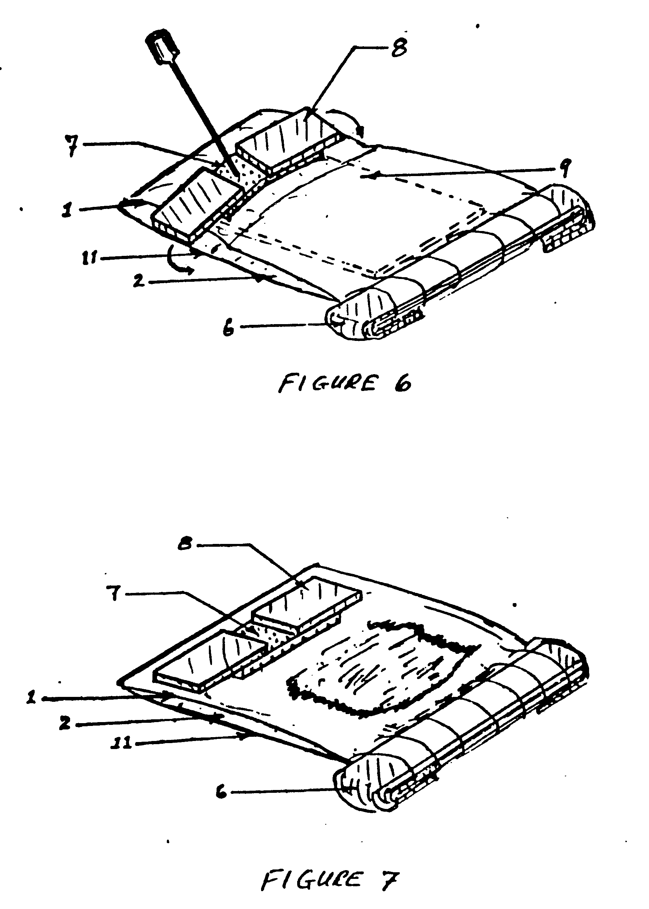

To initiate the test, the bottom part of the pouch is rolled forward firmly on the aluminum foil strips (Figure 4). This action increases the pressure within the diluent chamber, causing the lower frangible seal to rupture, and admitting the diluent to the reagent (dry component) chamber, where the thromboplastin and calcium are immediately dissolved. Further forward rolling of the pouch causes the rupture of the remaining frangible seal, admitting the solution to the viewing chamber. The ends of the foil strips then are bent under the roll (Figure 5) to fix the roll in place. The 0.5 ml. of fresh whole blood, or blood treated with sodium citrate (or other anticoagulants) is injected into the viewing chamber. The injection process is greatly facilitated by manipulation of the injection assembly, which is used to separate the front and back leaves of the chamber. When the pouch is held in the hand, the lateral ends of the levers can be pressed inwards and downwards. The lateral edges of the back plate act as fulcrums raising the medial ends of the levers, and with them the latex membrane, which in turn raises and separates the front leaf of the pouch from the back leaf. This action also stretches the latex membrane slightly near its mid-line. A hypodermic needle then is introduced through the latex into the reaction chamber (Figure 6), and the blood to be tested is injected. The needle is then removed immediately, a stop watch timer activated, and the pouch rotated back and forth gently but continuously. As soon as a fibrin web is observed (Figure 7), the time is recorded (normally about 60 seconds). A few seconds later an actual clot occurs. Blood exhibiting an abnormal prothrombin usually requires a period much longer than 60 seconds to begin to clot under these conditions.

Moreover, the only equipment necessary for obtaining accurate results is an hypodermic syringe and a watch with a second hand. No laboratory is required, nor is a laboratory professional needed, since the difference in clotting times for normal and abnormal blood is very large relative to those obtained using other procedures, and therefore easier to observe.

The injection assembly for the introduction of fluids into an otherwise nearly closed flexible chamber has several advantages. First, a space several mm. wide is created between the leaves of the chamber, making it more difficult to pierce the back leaf.

Second, the back plate, besides providing a fulcrum, also shields the back leaf from the needle. Thus, with reasonable care, needle puncture leaks in the pouch will not occur. Third, the hole produced by the hypodermic needle in the stretched latex membrane during injection becomes much smaller after the levers are released, thus cooperating with the natural elasticity of the rubber in the prevention of leaks through the needle track in the latex. Fourth, the specimen may be introduced into the chamber even when fluid is already present therein.

The diluent chamber also contributes to the utility of the device. Preferably, the volume of diluent in the diluent chamber is at least four times the volume of the sample itself. The dilution of the blood lengthens the clotting, or other reactions, reducing percentage error in the measurement of the time interval.

While in the example given above, the dry component prothrombin was diluted, and admitted to the viewing chamber before the specimen was injected, the device can easily be used in inverse manner, i.e., first injecting the specimen, then exposing it to the reagent. The latter approach is recommended in the case where APTT reagent is used in the measurement of activated partial thromboplastin time (APTT).

There may be more than a single reagent included in the bag, and the reagents need not all be disposed in the reagent chamber. The reagents may include dry components unstable in solution, such as the prothrombin of the example above, or they may be solutions. The reagents may be immediately released into the viewing chamber after rupture of a seal, or their release may be prolonged over time. Alternatively, reagents may already be present in the viewing chamber, as intimated above.

The pouch may be of various dimensions and constructed of a variety of materials such as Polyvinyl chloride and SURLYN (Trade Mark). The dry and liquid components can be used in varying quantities and can be varying composition. For example, instead of thromboplastin, other clot-inducing substances may be used: thrombin reagent may be used to measure "thrombin" time, or activated partial thromboplastin time (APTT) reagent may be used to measure APTT. The diluent may contain various buffering and stabilizing agents. Coloring agents (e.g. stains or dyes) or shaded backings may be employed to enhance clot visualization. Additional compartments may be constructed in the device to include different material in the reaction. For example, an additional compartment containing a blood serum known to be deficient only in a specific clotting factor may be added to the reaction to test for the presence of that factor in a test sample. If all reagents used are stable in solution, then the reagent chamber and the diluent chamber may be the same chamber. Samples other than blood may be tested for physical responses to chemical additives. For example, Limulus amebocyte lysate (LAL), in conjunction with cations and clotting proteins and enzymes, may be used in the gel-clot LAL test for endotoxins or pyrogens. LAL tests are summarized in T.J. Novitsky, LAL Methodology: the Choice Is Yours, Medical Devices and Diagnostics Industry, 49 (January 1984), incorporated by reference herein.

Figures 8-10 present views of the upper portion of a second embodiment in which Figure 8 is a front view, Figure 9 is a sagittal section, and Figure 10 is a cross section at the level of the back plate.

An elongated narrow aliquoting channel (14) is formed by a section of transparent film (15) being attached by a permanent seal (5) to the under surface of the front leaf, except at the distal end of the aliquoting channel the attachment is a non-permanent frangible seal (10). The back plate (9) is reduced in size from that of the preferred embodiment, and instead of being attached to the back leaf is attached to the under surface of the upper portion of the transparent film. The aliquoting channel when full contains a defined quantity of liquid, e.g. 9.5 ml.

To use this modification, blood is injected through the latex membrane into the aliquoting channel. When the channel is full, the needle is withdrawn and the frangible seal ruptured by external pressure over the channel. The use otherwise does not differ from that of the preferred embodiment.

- 12 -

The advantage of this modification is that it allows a measured quantity of blood to be utilized in the device without needing to resort to external means for measurement.

It should be evident that further flexibility is possible if there are additional frangible seals at predetermined points along the length of the channel.

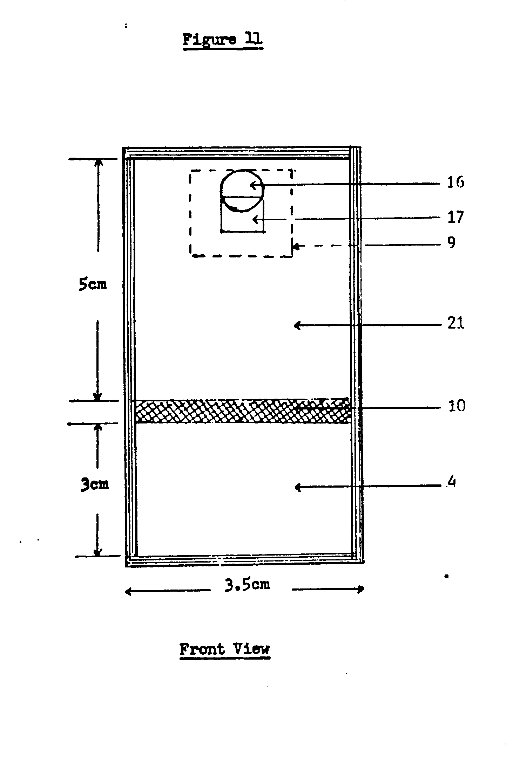

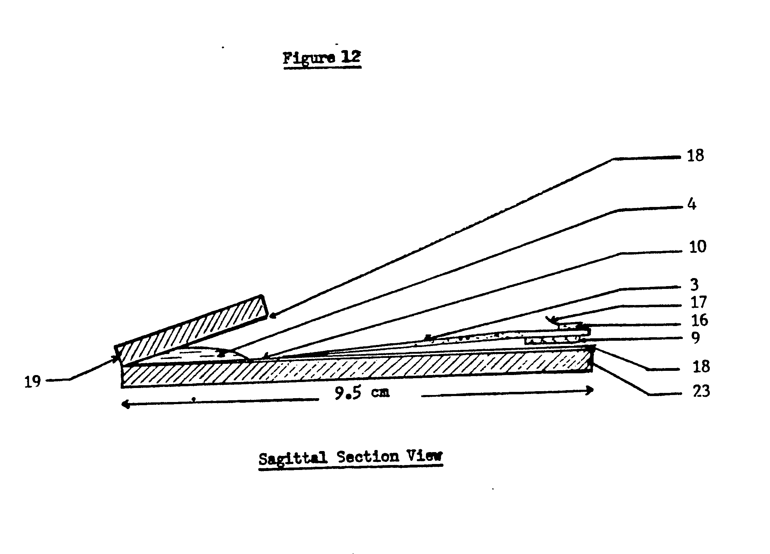

A third embodiment appears in Figures 11 and 12. Figure 11 shows the front surface of a two chamber specimen bag. The diluent chamber 4 contains liquid and substances (such as latex particles) which may be stored in liquid for later use. The dry reagent chamber 21 contains substances which deteriorate or inappropriately react when stored in liquid form, for example lyophilized thrombin or thromboplastin. The reagent chamber also serves as a viewing chamber. The two chambers are separated by a frangible seal 10. The viewing and reagent chamber 21 has attachments to its under surface and its upper surface. Attached to the outside of the upper surface is an elastic membrane 16 which stretches and which closes after itself after being punctured, for example latex or rubber. Attached to this self-sealing septum is a pull tab 17 made of any convenient material, such as transparent polycarbonate. The reinforcing plate 9 attached to the outside of the undersurface consists of any hard material such as PVC or polycarbonate. Preparation of the device for sample testing consists of mixing the contents of the diluent chamber with other contents of the viewing chamber. The frangible seal 10 separating the two compartments is opened by increasing internal pressure between the upper and under sides of the bag. The internal pressure may be increased in the diluent chamber by applying external pressure, by squeezing with the fingers, pressing between the hand and a hard surface, or by rolling upward the bottom edge of the bag, compressing fluid against the edge of the frangible seal.

After breaking the frangible seal, the diluent reagent and dry reagent are mixed. A gaseous bubble previously introduced into one or both chambers may be used as a stirring means to facilitate agitation and mixing. Nearly all of the diluent material may be moved into the dry reagent chamber by rolling or other external pressure applied to the diluent chamber. Continued exclusion of reagent from the diluent chamber permits subsequent analysis to be confined to the dry reagent chamber. Alternatively, the frangible seal may be made to open broadly to allow access of reagent to both chambers so that subsequent analysis may occur in either one or both chambers simultaneously.

A test sample (or other substance) is introduced into the analysis bag through the membrane 16 by injection after pulling apart the under and upper sides of the bag by pulling outward on the pull tab and applying an opposing resistance to the reinforcing plate. The reinforcing plate may be small, as in figure 11, or may be larger, forming a flat surface acting as a backing of the device, or even larger than the bag itself. It is rigged to resist the force exerted through the pull tab, and its surface is hard to resist puncture by and to deflect a hypodermic needle.

The opposing forces applied through the pull tab and the reinforcing plate separate the sides of the bag to form a vacant tent even if air has been evacuated from the dry reagent chamber. The force required to form the tent also stretches the elastic membrane 16 and pulls the underlying upper side of the bag taut which facilitates puncture by a piercing means (e.g., hypodermic needle). If the piercing means continues its traverse through to the under side of the bag, then its penetration of the under side is prevented and deflected by the hard surface of the reinforcing plate. A test sample (or other substance) is injected into the analysis bag through the pointed instrument. When the piercing means is withdrawn, the elastic membrane seals behind it and prevents leakage from the bag.

Additionally, other chambers than that illustrated may be modified to facilitate the receipt of a sample. The order of introduction of the sample and the mixing of reagents, diluents, etc. held in the bag may be varied as desired for particular specimen preservation or analysis.

When the dry reagent is unstable, use of the two-chambered bag requires that the liquid and dry reagents be mixed prior to injection of the sample, and the timing of the reaction initiated with the injection of the sample. External pressure is applied to the diluent chamber to break the frangible seal allowing access of the liquid to the dry reagent. Gentle massage of the reagents through the pouch facilitates mixing. A selected quantity of air (or some other gas such as nitrogen) may be included in the diluent or dry reagent chamber to form a bubble in the device. The bubble is a mechanical aid to mixing the reagents or to mixing the reagent with the sample. The bubble may be massaged or the device may be rotated to produce movement of the bubble to increase agitation of the solution. The mixing of reagents should be complete prior to addition of the test sample. This requires about 5 seconds for thrombin, thromboplastin or partial thromboplastin and saline in the clotting time experiments.

The device is prepared for receiving the sample by pulling on the pull tab and causing the reinforcing plate to resist that force. The opposing forces may be applied by holding the pull tab in one hand and the reinforcing plate in the other. Afterward the tent shape may be maintained with one hand, while the other is used to inject a specimen. If the reinforcing plate is made sufficiently large, or attached to a sufficiently large backing, then the little finger or flat of the hand may be rested on it to hold it in place as the pull tab is grasped between thumb and forefinger and pulled in order to expand the pouch.

A hypodermic needle easily penetrates the stretched elastic membrane and pouch and facilitates specimen injection. The pull tab is released after the needle is inserted into the pouch and the hand formerly holding the pull tab used to start a timer as the specimen is injected. The needle is removed and the device gently rotated to promote mixing. Inclusion of an air bubble as an internal stirring means promotes this mixing. The timer is stopped when the reaction appears (e.g. fibrin web forms).

Figure 12 is a sagittal view that helps visualize a means constructed to aid the operation of the two chambered pouch.

Figure 12 also shows a two chambered pouch fixed to an external compression means prior to use. The compression means preferably consists of two rigid or semi-rigid panels 23 and 19. The width of the compression means should be equal to or greater than the width of the pouch. The length of the panel underlying the combined viewing and dry reagent chamber should be as long as or longer than that chamber. The extent of other panel should be equal to or greater than the comparable extent of the diluent chamber. The two panels are hinged at their upper surfaces, facing the pouch, so that they may be folded over one another and their opposed surfaces fit flush to one another when folded. The hinges may be formed of a strip or sheet of fabric or plastic 18 adhered to the upper surface of the panels or by recessed metal or plastic hinges. The hinged space between the panel underlying the combined dry reagent and viewing chamber and the panel overlying the diluent chamber should underlie the bottom of the pouch. The lowermost panel is folded over the diluent chamber to rest against its upper surface.

The upper surface of the operating means is coated partially or entirely with adhesive (e.g. liquid glue or two-sided tape) sufficiently tacky to hold the pouch in place, and to hold the panels flush to one another when folded, but also sufficiently loosely adhesive to permit unfolding of the panels and removal of the disposable plastic pouch. Several pouches may be used in succession before requiring re-application of adhesive, and the hinged panels used through many applications of adhesive before requiring replacement. Since the hinged panel compression means is to be used many times it may be made of metal, wood, glass or plastic and still be economical, or it may be made of cardboard.

Use of the compression means required placement of a disposable analysis pouch on the upper surface of the hinged panels so that the frangible seal overlies the hinged space. The bottom panel is folded over the top of the diluent chamber and pressed down so that the frangible seal breaks the diluent into the dry reagent. Pressing the two panels enclosing

- 16 -

the diluent chamber tightly together ensures expulsion of diluent from its chamber and opposition of the two adhesive surfaces of those panels insures prevention of the diluent from returning into the chamber.

A locking mechanism to hold the two panels together without the use of adhesive also works well (though it is not illustrated), but adhesive is advantageous in holding the pouch in place during operation.

The back plate underlying the viewing chamber may be colored or transparent, plastic or other material. It should be sufficiently rigid to hold or to act as the reinforcing plate 9 in order to resist the force of pulling on the tab in preparation for specimen injection.

In a fourth embodiment, the "pull tab expander" injection means is adapted to a three chamber analysis bag. The main benefit of a three chambered device is that a sample may be injected into the viewing chamber 2 and, subsequently, the timing of the reaction initiated as the previously mixed liquid and dry reagents are forced into the viewing chamber.

The three chambered analysis bag is used by breaking the frangible seal 10 between the diluent chamber 14 and dry reagent chamber 3, and as the two reagents are mixing injecting a sample into the viewing chamber 2 by way of the elastic membrane 16 stretched between the pull tab 17 and the reinforcing plate 9. Timing of the reaction begins as the mixed liquid and dry reagents are expelled into the viewing chamber. Expulsion of the reagents is easily accomplished by rolling the bottom of the pouch into a coil which gradually increases internal pressure of the reagents against the frangible seal leading to the viewing chamber.

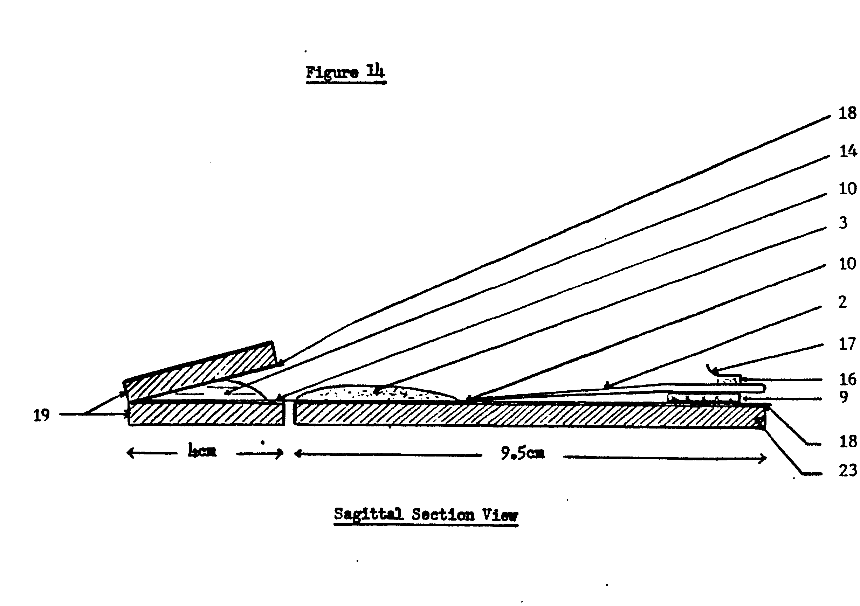

Figure 14 shows the adaptation of the hinged panel means to a three chambered analysis bag. The compression means differs from that shown in figure 12 in that a middle hinged panel 19 was added. The analysis bag is fitted to the compression means so that the frangible seal 10 separating the diluent chamber 14 and dry reagent chamber 3 overlies the top hinged edge of the middle panel, and the bottom of the analysis bag ends at the bottom edge of the middle panel. The bottom panel is folded over to rest on the top of the diluent chamber. The middle and the bottom panels are of the same width as the diluent chamber so that the top edge of the middle panel meet at the frangible seal when the two panels are folded closed during operation.

The hinged gap between the panel underlying the viewing chamber and middle panel is equal to the thickness of the bottom panel. The middle and bottom panels with the diluent chamber 14 tightly interposed between them, may then be folded over the dry reagent chamber and pressed flush to the surface of the panel underlying the viewing chamber. The opposing surfaces of the three panels, as they are folded over one another, are coated with adhesive so that they maintain the intervening chamber in a compressed state after pressure is released. This means that reagent does not return to chambers from which it has been expelled.

In clotting time tests the operator may choose first to inject a specimen into the viewing chamber 2, or to first expel the diluent into the dry reagent chamber 3 by compressing the bottom and middle hinged panels 19 together. The operator may also expel the mixed reagents into the viewing chamber by folding and pressing the compressed panels over the dry reagent chamber either before or after injection of the specimen. Timing of the reaction begins as the reagents and specimen combine.

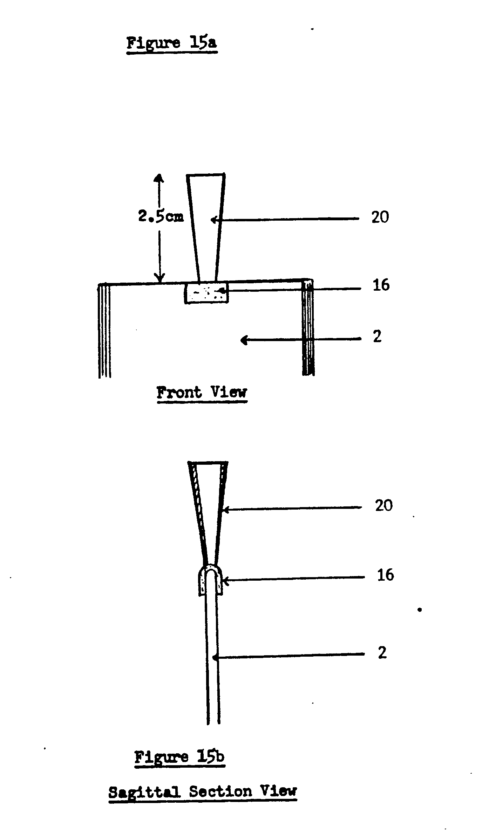

Figure 15 shows another adaptation of the elastic membrane injection assembly. Comparison of a front view (a) and a sagittal view (b) shows that the elastic membrane 16 is adhered to the pouch around one edge. The membrane is attached for injection into the viewing chamber in this case, but may be located on other chambers if desired. No further attachment is required for a self-sealing injection port. The elastic membrane and enclosed edge are held firmly in place as a piercing means (e.g. hypodermic needle) is inserted and withdrawn through them so that its trajectory is parallel to the flat sides of the chamber. Care must be taken to keep the point's trajectory parallel to prevent punctures of the flat sides of the pouch. The funnel (or tube) guide 20 shown attached to the elastic membrane 16 acts as a guide for easy access to the membrane and to maintain the point's trajectory parallel to the pouches sides. Also the length of the funnel is selected so as to limit the distance the puncturing means may be inserted before the main body of the injection instrument (e.g. syringe barrel) is blocked by the mouth of the funnel. In use, the funnel is held firmly in place as the injection instrument point is inserted into the wide mouth of the funnel. The injection instrument then is pushed toward the pouch until stopped by the mouth of the funnel, at which time the injection instrument's point is automatically correctly positioned within the chamber for injection.

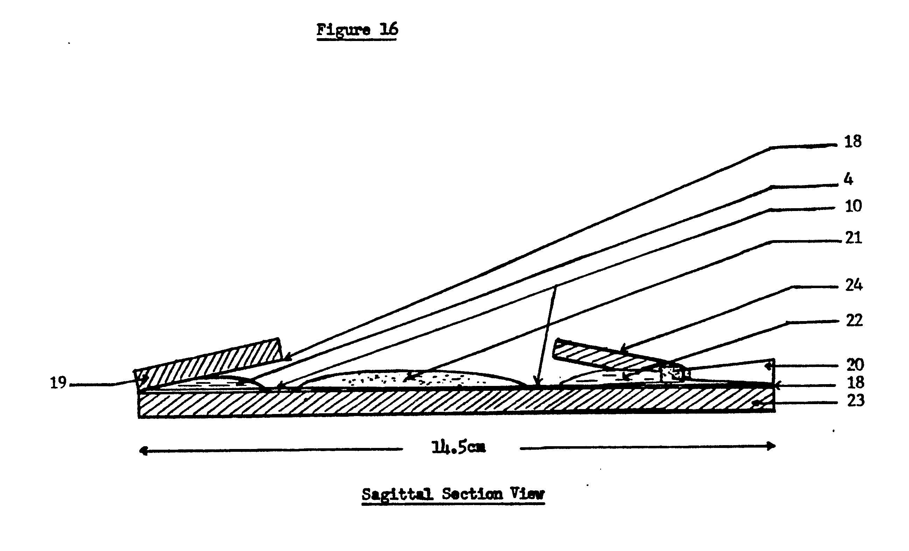

Figure 16 shows a three chambered analysis bag in which the central chamber contains the dry reagent and is also the viewing chamber 21. The means of operation of the bag is also shown, but the disposable pouch containing all necessary reagents consists of a diluent chamber 4 separated by a frangible seal 10 from the central viewing chamber 21 which also contains dry reagent and a specimen chamber 22 which has an elastic membrane 1 (not shown) attached to its top edge as in figure 5. The specimen compartment is constructed to hold a predetermined volume (e.g. 0.5 ml of blood) when full. Air is evacuated from the chamber during construction by, for example, inserting a hypodermic needle attached to a syringe and extracting the gas. The specimen may be introduced using any sharp pointed injection instrument. After the point penetrates the elastic membrane into the pouch, injection is continued until strong resistance is met and the chamber appears full, at which time the volume of specimen is the predetermined volume of the compartment. The diluent compartment then is compressed breaking the liquid across the frangible seal to mix with the dry reagent. The bottom edge of the diluent compartment may be coiled up to the frangible seal to exclude diluent from returning to that compartment. Timing of the

- 19 -

reaction is initiated as the specimen compartment 22 is compressed, breaking the specimen into the central viewing chamber 21, and the bottom edge of the chamber may also be solid to prevent backflow. Compression may be achieved by a hinged panel device such as that taught above or by other means such as squeezing, rolling or winding.

The expansion/compression means shown in Figure 16 may be constructed of highly durable materials (e.g. plastic, wood, or metal) and reused many times with a succession of disposable analysis bags. The compression means consists of a back panel 23 with a funnel guide adhered to it at one end and hinged panels attached at other end of the back panel 19 and adjacent to the narrow end of the funnel 24. The opposing surfaces of the panels are coated with a long lasting and renewable tacky substance. An analysis bag is placed on and adhered to the panels so that its elastic membrane 16 lies immediately adjacent to the narrow end of the funnel guide and its bottom end lies at the point of flexion of the hinge sheet 18. The hinged panel 24 is pressed down on the evacuated specimen compartment (22) so the top of the pouch adheres. When the expansion/compression panel is raised the top of the pouch rises with it to form a large vacant tent to accommodate the insertion of pointed objects into the specimen compartment. Also, the hard surfaces of the panels deflect and prevent puncture by pointed objects thereby preventing leaks. The injector means may be quickly and easily inserted into the funnel guide 20 and into the pouch. Expansion of the evacuated specimen compartment also aids rapid filling of the specimen compartment to the predetermined volume. The diluent is broken across the lower frangible seal 10 to mix with the dry reagent before the specimen is broken across the upper frangible seal 10 into the same central chamber. Timing is initiated as specimen and reagents are mixed.

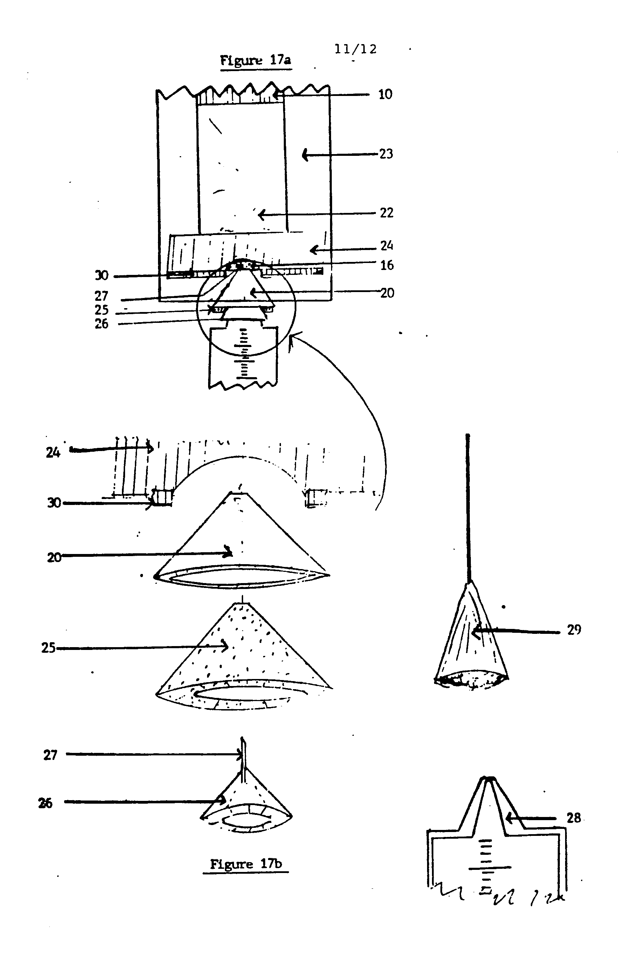

Figure 17 illustrates a permanent and reusable means for injection of substances into disposable flexible pouches through an attached elastic membrane port. Its configuration during operation (a) and its components (b) are shown. In Figure 17a, a disposable flexible bag (e.g. made of plastic film or rubber) is adhered to a back panel 23 and expansion/compression panel 24 as described for Figure 16. Also shown is a fulcrum/backstop 30 fixed to the back panel 23 and hinged to the expansion compression panel 24 which functions as the point of pivot as the panel 24 is pulled upward and as a backstop as it is pushed downward. Figure 17a shows a funnel guide 20 permanently fixed to a back panel 23 also as described for Figure 16, but widened to contain several inserted components. The inserted components are a set of funnels and cones of various sizes which fit one into another as illustrated in Figure 17b. A compression cushion 25, made of soft material such as sponge or neoprene rubber, is permanently fixed into the hollow center of the funnel guide 20. The shape of the compression cushion 25 is a hollow cone (that is, a closed tip or apex whereas the funnel shapes have open tips) with outside wall dimensions identical to the wall dimensions of the inside hollow of the funnel guide 20 within which it fits. A hollow friction-fit funnel 26 with attached hollow needle 27 is fixed inside the hollow of the compression cushion 25. The friction-fit funnel 26 is made of hard durable material such as plastic, metal or wood, as is the attached hollow needle. The hollow of the friction-fit cone is shaped and roughened to fit by friction to the tapered end of a sample delivery system 28 such as a syringe as illustrated or pipette (not illustrated). The sample delivery system is not an integral part of the injector system but is provided separately. The two funnels 20 and 26 are permanently fixed around the central compression cushion 25. The hollow needle 27 attached at the apex of the friction-fit funnel 26 extends into but does not exit from the apex of compression cushion 25 when the system is at rest. However, when pressure is applied to the back of the friction-fit cone, as when a syringe is pushed hard into its center, the compression cushion 25 is compressed to become thinner and the tip of the hollow needle pushes through the cushion and open end of the funnel guide 20 into the elastic membrane and analysis pouch. An obturation 29 is inserted in place of a sample delivery system when the injector system is not in use to keep the injection pathway clear.

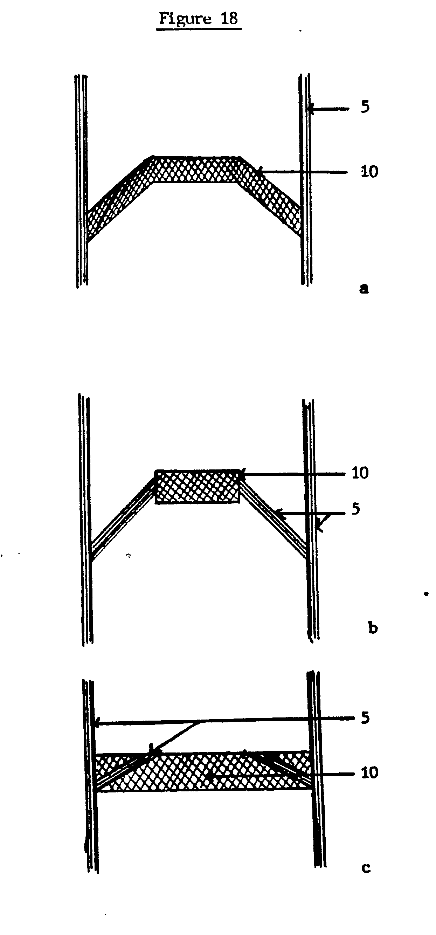

Figure 18 illustrates expanded views of some variations in construction of frangible seals to direct the flow of liquid from a lower compartment into an upper compartment. Figure 18a shows permanently sealed edges 5 of the pouch and a trapezoidal frangible seal 10 forming the separation between an upper and a lower compartment. The frangible seal has two oblique side segments and a central horizontal segment. External pressure applied to liquid in the lower compartment is directed upwards concentrated at the center of the frangible seal making the center most likely to break open to allow flow of the liquid into the upper compartment. This creates a large break which permits smooth and rapid mixing of the contents of the two compartments. If, external pressure is instead applied to the upper compartment, it is directed mainly at the points of attachment of the frangible seal to the compartment walls, where breakage is less likely to occur.

A more secure configuration is shown in figure 18b, in which oblique permanent seals replace the obliquely oriented frangible seal side segments. With the configuration of Figure 18b, flow from the lower to the upper compartment is very likely and flow from the upper to the lower is very unlikely when appropriate external pressures are applied. The configuration of figure 18c functions comparably to that shown in 18b but is more convenient to manufacture. In this configuration, the central segment is extended to contact the permanently sealed edges of the pouch.

- 22 -

C L A I M S

1. A specimen bag comprising at least one chamber having an injection assembly whereby a substance may be injected into the collection chamber, said injection assembly comprising an elastic septum and means for stretching said septum.

2. A bag according to claim 1, wherein said stretching means comprises at least one lever and a fulcrum about which said at least one lever may operate.

3. A bag according to claim 2, further comprising backing means attached to said bag on the side opposite said septum means, said backing means being so positioned that at least one edge of said backing means may act as said fulcrum.

4. A bag according to claim 3, wherein said backing means is attached to the interior of said bag, whereby said backing means shields the side of the bag opposite said septum means from puncture.

5. A bag according to any one of the preceding claims, wherein said stretching means comprises a pull tab attached to said septum, and means for exerting a force opposing that exertable through said pull tab.

6. A bag according to any one of the preceding claims including funnel guide means for guiding an injector to the septum and for limiting its penetration.

7. A bag according to claim 6, wherein said guide means guides the injector along the longest axis of said bag.

8. A bag according to any one of claims 1 to 6, having a plurality of said chambers, with adjacent communicating said chambers separated by independently frangible seals.

9. A bag according to claim 8, and further comprising external compression means whereby pressure may be exerted on a first said chamber such that an adjacent frangible seal is broken and the contents of said chamber forced into a second adjacent said chamber.

10. A bag according to claim 9, wherein said external compression means comprise a fixed panel affixed to the wall of said chamber, a movable panel, and a hinge articulably connecting said movable panel to said fixed panel such that said chamber lies between said panels.

11. A bag according to claim 9 or 10, further comprising adhesive means releasably affixing said compression means to said bag about said first chamber, said compression means being further adapted as expansion means for expanding said chamber to receive a substance.

12. A bag according to any one of claims 8 to 11, further comprising rolling means affixed to the distal end of the bag about which the bag may be rolled toward the proximal end, thereby rupturing the frangible seals of the bag in the proper order.

13. A bag according to claim 12, wherein said rolling means is made of a flexible material, and extends beyond the edges of the bag, whereby the overhanging ends of the rolling means may be folded over to prevent the bag from unrolling.

14. A bag according to any one of claims 8 to 13, wherein said frangible sealing means are adapted to be easily broken when external pressure is exerted on the chamber to one side of said seal but not easily broken when external pressure is exerted on the chamber to the other side of the seal.

15. A bag according to claim 14, also comprising a third compartment adjacent to the first compartment and separated therefrom by a second frangible sealing means, which second means is not easily broken when pressure is exerted on the first compartment.

16. A bag according to any one of claims 8 to 15, wherein said chambers include a collection chamber, a dry reagent chamber, and a diluent chamber, said dry reagent chamber being chargeable with a dry reagent which is unstable in solution.

17. A bag according to claim 16, wherein the reagent comprises Limulus amebocyte lysate.

18. A bag according to claim 17, in which each said frangible seal is of a substantially trapezoidal shape tapering in the desired direction of fluid flow.

19. A bag according to any one of claims 16 to 18, in which either the reagent or the diluent chamber contains bubble means whereby the diluted reagent may be stirred after the frangible seals between said chambers are broken.

20. A bag according to any one of claims 1 to 19 and further comprising an aliquoting channel having a proximal end and a distal end, communicating at the proximal end with said septum, and at the distal end with said chamber, said septum not communicating directly with said chamber, said aliquoting channel having at least one frangible seal along its length, and the volume defined by the proximal end of the aliquoting channel and said channel seal being predetermined.

21. A bag according to claim 20, wherein said aliquoting channel has a frangible seal at the distal end.

22. A bag according to any one of claims 1 to 4, further comprising means for expanding said chamber prior to injection.

23. An injection means for a specimen bag comprising a pierceable, resealing elastic septum; a tapering guide means having its narrow end communicating with said septum; a compressible cushion affixed to the inner surface of said guide means so as to fit therewithin, said funnel means having hollow piercing means extending into said passageway from the narrow end of said funnel means, and said piercing means being capable of piercing said septum means only when pressure is applied to said funnel means urging it in the direction of said septum, whereby said cushion means is compressed and said piercing means brought within reach of said septum.

24. A specimen collection or analysis bag having at least two chambers separated by a frangible sealing means, said sealing means having a substantially trapezoidal shape.

25. A specimen collection or analysis bag comprising an upper sheet and a lower sheet, and a peripheral seal sealing said lower sheet to said upper sheet to define a space, and septum means within said peripheral seal communicating with said space.

26. A specimen bag comprising at least three compartments and independently frangible sealing means separating at least two pairs of adjacent compartments, said frangible sealing means being adapted to being more easily broken in a predetermined sequence, and wherein one of said compartments is adapted to receiving a specimen.

27. A specimen bag comprising an injection assembly whereby a substance may be injected into the chamber, said injection assembly comprising elastic septum means and funnel guide means for guiding an injector to the septum and limiting its penetration.

FIGURE 1

FIGURE 2

FIGURE 3

0157579

2/12

FIGURE 4

FIGURE 5

FIGURE 6

FIGURE 7

FIGURE 8

FIGURE 9

FIGURE 10

5/12

## Figure 11

16
17
9
5cm
21
10
3cm
4
3.5cm

## Front View

Figure 12

18
4
10
3
17
16
9
18
23

19

9.5 cm

Sagittal Section View

6/12

0157579

**Figure 13**

0157579

16

17

9

5cm

2

10

3.5cm

3

10

3cm

4

←——3.5cm——→

**Front View**

## Figure 14

18
14
10
3
10
2
17
16
9
18
23

19

|← 4cm →| |← 9.5cm →|

**Sagittal Section View**

Figure 15a

2.5cm — 20

16

2

Front View

20

16

2

Figure 15b

Sagittal Section View

## Figure 16

18
4
10
21
24
22
20
18
23

19

14.5cm

Sagittal Section View

## Figure 17a

10
23
22
24
16
30
27
25
26
20

24
30
20
25

27
26

## Figure 17b

29

28

Figure 18